# EUROPEAN PATENT SPECIFICATION

(11) **EP 2 100 629 B1**
(45) Date of publication and mention of the grant of the patent: **22.05.2013**
(21) Application number: 09003409.1
(22) Date of filing: 09.03.2009
(51) Int. Cl.: A61L 27/38

(54) **Bone tissue regeneration sheet and production method thereof**
Knochengeweberegenerierungsfolie und Herstellungsverfahren dafür
Feuille de régénération de tissu osseux et son procédé de production

(30) Priority: 10.03.2008 JP 2008059871
(43) Date of publication of application: 16.09.2009
(73) Proprietor: GC Corporation, Itabashi-ku Tokyo 174-8585 (JP)
(72) Inventor: Yamanaka, Katsuyuki, Tokyo 174-8585 (JP); Yamamoto, Katsushi, Tokyo 174-8585 (JP); Suda, Youko, Tokyo 174-8585 (JP); Sakai, Yuhiro, Tokyo 174-8585 (JP); Kaneko, Tadashi, Tokyo 174-8585 (JP)
(74) Representative: Müller-Boré & Partner Patentanwälte

(56) References cited:
- EP-A- 0 798 374
- EP-A- 1 649 872
- US-A- 5 944 754
- MACKIE E J ET AL: "Endochondral ossification: How cartilage is converted into bone in the developing skeleton" INTERNATIONAL JOURNAL OF BIOCHEMISTRY AND CELL BIOLOGY, EXETER, GB, [Online] vol. 40, no. 1, 29 June 2007 (2007-06-29), pages 46-62, XP022613249 ISSN: 1357-2725 [retrieved on 2008-08-05]

## Description

The present invention relates to a bone tissue regeneration sheet material used for repairing a bone defect caused by a disease or an accident such as a bone tumor extirpation or a fracture, and also relates to a production method thereof.

A regenerative medicine for biotissues and internal organs, which lapse into dysfunction or malfunction, is required now. The regenerative medicine is a new medical technique for regenerating a shape and a function of a biotissue similar to those of an original biotissue, which cannot be repaired by a healing capability inherently equipped in a living body, by using three factors; cell, carrier, and growth factor.

A bone substitute is used for inducing a bone regeneration, and the bone substitute is made of hydroxyapatite or tricalcium phosphate. However, since it is preferable that foreign matters do not remain in a living body, a matrix is required to have biocompatibility or bioabsorbability. For example, in Japanese Patent Application Laid-Open No. 2006-116212, a bioabsorbable synthetic high polymer, such as polyglycolic acid, polylactic acid, or a copolymer of glycolic acid and lactic acid, is used as a matrix of a bone substitute. Further, in Japanese Patent Application Laid-Open No. 2004-105046, a porous carrier material prepared by a natural polymer such as gelatin is used as a matrix of a bone substitute. As for an example in which a matrix is applied to a regeneration inductive sheet for a bone tissue, Japanese Patent Application Laid-Open No. 2004-24706 discloses a sheet produced by sandwiching tricalcium phosphate between sheets which are softer than tricalcium phosphate to make a laminate. However, this method has a deficit that toughness is insufficient for a bone since tricalcium phosphate is used.

After these developments, a bone regeneration sheet made of a matrix having cultured cells was developed. For example, Japanese Patent Application Laid-Open No. 2003-275294 proposes a bone regeneration sheet produced by laminating a cultured cell sheet and a biodegradable sheet, where the cultured cell sheet is made by culturing mesenchymal stem cells in a sheet shape, and the biodegradable sheet is made by forming a biodegradable material in a sheet shape. Further, for example, Japanese Patent Application Laid-Open No. 2006-116212 also discloses a mesenchymal tissue regeneration inductive sheet produced by adhering mesenchymal tissue precursor cells and extracellular matrix on a porous sheet, where the mesenchymal tissue precursor cells are differentiated from a mesenchymal tissue. In these inventions, a sheet to which cultured osteoblasts are adhered is taken into a living body, and a cortical bone is formed from the osteoblasts by a membranous ossification in the living body. However, since there is a problem that osteoblast-like cells cannot be cultured in multilayer, a regeneration sheet, in which an osteoblast layer is used and the cell layer has a thickness of 100µm or more, can not be provided.

The present invention is directed to provide a bone tissue regeneration sheet having a thick cortical bone cell layer which cannot be formed on a conventional bone like tissue regeneration sheet using cultured osteoblasts, and a production method thereof.

Present inventors carried out earnest works to solve the aforementioned problems and, as a result, they found out the followings to complete the present invention: A bone tissue regeneration sheet having a multi cell layer of 200µm or more in thickness can be made by once forming a cartilaginous tissue capable of forming a thick cell layer on a high polymer sheet, instead of a conventional bone tissue regeneration sheet using cultured osteoblasts, and making the cartilaginous tissue to be cortical bone cells (more particularly, cortical bone-like and/or calcified cartilage-like bone cells made by culturing) via endochondral ossification.

According to an aspect of the present invention, a production method of a bone tissue regeneration sheet includes steps of forming a chondrocyte layer having a thickness of 200µm or more on one side of a porous bioabsorbable polymer sheet, and making endochondral ossification of the chondrocytes so as to form a cortical bone tissue layer having a thickness of 200µm or more.

In the production method of a bone tissue regeneration sheet, a method for forming a chondrocyte layer having a thickness of 200µm or more on one side of a porous bioabsorbable polymer sheet includes steps of seeding chondroblasts or stem cells for differentiating to chondrocytes on one side of a porous bioabsorbable polymer sheet, taking the cell seeded sheet into a culture medium, applying centrifugal force of 100 to 1000G to the culture medium for a predetermined time so as to aggregate the chondroblasts or the stem cells for differentiating to chondrocytes, and culturing the aggregated cells in a culture medium not containing serum but containing one or two or more kinds selected from ascorbic acid, an ascorbic acid derivative, and dexamethasone without applying centrifugal force to the culture medium so as to form a chondrocyte layer having a thickness of 200µm or more. A method for making endochondral ossification of the chondrocytes so as to form a cortical bone tissue layer having a thickness of 200µm or more includes a step of culturing the chondrocyte layer of 200µm or more in a culture medium containing serum so as to make endochondral ossification of the chondrocyte layer to form a cortical bone tissue layer of 200µm or more.

According to another aspect of the present invention, a bone tissue regeneration sheet has a cortical bone tissue having a thickness of 200µm or more on one side of a porous bioabsorbable polymer sheet, and is obtainable by the production method of a bone tissue regeneration sheet above.

The bone tissue regeneration sheet according to the present invention has a thickness of a bone cell layer of 200µm or more and is capable of shortening a healing time and improving strength of the bone tissue regeneration sheet. Thus, the bone tissue regeneration sheet is more excellent than a conventional bone tissue regeneration sheet using osteoblasts. Such a thick bone cell layer becomes possible to be formed by a similar reaction to that of endochondral ossification of the chondrocyte layer having a thickness of 200µm or more formed on one side of the porous bioabsorbable polymer sheet.

A bioabsorbable polymer constituting a sheet-shaped porous body used in the present invention is a bioabsorbable polymer selected, for example, from at least one kind of homopolymer or copolymer selected from polyglycolic acid, polylactic acid, poly-ε-caprolactone, polyamino acid, polyorthoester, polymalic acid, and a copolymer of those. The bioabsorbable polymer has high strength and has safety to a living body, so it is preferable.

Further, the bioabsorbable polymer has preferably a molecular weight of 10,000 to 500,000. If the molecular weight is less than 10,000, the strength of the sheet may decrease. If the molecular weight is more than 500,000, the sheet is too hard, so it is hardly used. Further, the sheet of a bioabsorbable polymer has necessarily a porous body, and has preferably a pore diameter of 1 to 500µm. If the diameter is less than 1µm, the sheet has insufficient flexibility. If the diameter is more than 500µm, the sheet surface is too coarse, so that the seeding efficiency of cells may decrease.

As for the chondroblasts or the stem cells for differentiating to chondrocytes to be used in the present invention, the chondroblasts can be directly used, or stem cells capable of differentiating to chondrocytes, such as mesenchymal stem cells, mesenchymal cells, and synovial cells, or stem cells capable of accelerating repair of them can be used. As for these stem cells, for example, cells taken from marrow of a pelvis (an ilium) or a long tubular bone of hand and foot (a femur and a tibia), marrow of an alveolar bone, a periosteum, a synovial membrane, and/or a periosteum of a palate or an alveolar bone, can be used. As for a method for taking the cells, any method used in a medical department can be used without particular limitation. However, it is preferable that cells are taken from marrow of an ilium and the like or a periosteum of a palate, an alveolar bone or the like, because these cells are taken by a simple operation which requires only a minimum exfoliation and incision of the skin and muscle.

The taken chondroblasts or the stem cells for differentiating to chondrocytes are amplified and cultured in a culture dish for culturing a cell for 1 to 2 weeks by a general method. As for a culture medium used for culturing, a proper culture medium can be used. However, for example, a αMEM culture medium for culturing cells, which contains autologous serum or fetal bovine serum, can be properly used. At this time, if a specific growth factor (e.g. , bFGF) is acted to the mesenchymal stem cells, the mesenchymal stem cells are proliferated while holding high multi-differentiating potency so as to accelerate regeneration of a cartilage, and thus exercise remarkably regenerating potency.

The mesenchymal stem cells amplified and cultured while holding multi-differentiating potency under the existence of the specific growth factor are exfoliated from the culture dish by a treatment with trypsin or the like, and seeded on one side of the porous bioabsorbable polymer sheet.

The chondroblasts or the stem cells for differentiating to chondrocytes are seeded on one side of the porous bioabsorbable polymer sheet, taken into a culture medium, and then applied with centrifugal force of 100 to 1000G, preferably 200 to 600G, by a centrifugal separator or the like for a predetermined time. When once applying the centrifugal force to the stem cells, the stem cells can accurately differentiate to chondrocytes without culturing them under pressurization thereafter. Further, the chondroblasts or the stem cells for differentiating to chondrocytes can be invaded into the porous sheet under a high pressure by the centrifugal force. As a result, the thick chondroblast layer can be formed.

At this time, the centrifugal force can be applied in a direction from the seeded chondroblasts or stem cells for differentiating to chondrocytes toward the porous bioabsorbable polymer sheet, or the centrifugal force can be applied in a direction from the porous bioabsorbable polymer sheet toward the seeded chondroblasts or stem cells for differentiating to chondrocytes. However, when the centrifugal force is applied in the direction from the seeded chondroblasts or stem cells for differentiating to chondrocytes toward the porous bioabsorbable polymer sheet, the cells can be easily invaded into the porous sheet, so that high cell seeding efficiency can be obtained. Thus, it is preferable.

A time for applying the centrifugal force is changed according to the intensity of the centrifugal force or the condition of the porous bioabsorbable polymer sheet. However, the time within from 30 seconds to 30 minutes is preferable. Further, a temperature at the time of applying the centrifugal force is equal to a general cell culture temperature and is not restricted especially.

The centrifugal force of 100 to 1000G is applied to every culture medium including the porous bioabsorbable polymer sheet, where the chondroblasts or the stem cells for differentiating to chondrocytes are seeded, so as to aggregate the cells. Thereafter, the porous bioabsorbable polymer sheet is cultured for 3 to 4 weeks under ordinary pressure using a culture medium not containing serum (e.g., a culture medium disclosed in Science 284, 143-147, 1999) without applying centrifugal force, so as to culture or differentiate sufficient amount of chondrocytes on one side of the sheet. The final thickness of a cortical bone tissue can be controlled with the thickness of the chondrocytes at this time. The chondrocytes can form a thicker cell layer than that of osteoblasts and, more particularly, can form a cell layer having a thickness of 200µm or more. The culture medium contains necessarily one or two or more kinds selected from ascorbic acid, an ascorbic acid derivative, and dexamethasone. If the culture medium does not contain those, a chondrocyte layer having a sufficient thickness cannot be obtained.

The serum in the present invention is a supernatant fraction obtained by removing a hemocyte component from coagulated blood. For example, the serum is human serum, fetal bovine serum, or fetal horse serum.

The ascorbic acid derivative is, for example, ascorbic phosphate ester, ascorbic palmitate ester, ascorbic stearate ester, ascorbic acid glucoside, sodium ascorbate, ascorbic dipalmitate, ascorbic sulfate ester, or L-3-O-ethylascorbic acid. These ascorbic acid derivatives can be used independently or by mixing two or more kinds arbitrarily and used simultaneously with ascorbic acid and dexamethasone.

In the production method of the bone tissue regeneration sheet according to the present invention, the chondrocyte layer is once formed on one side of the polymer sheet by the aforementioned method, and thereafter the chondrocyte layer is made to a cortical bone tissue layer and/or calcified cartilage tissue layer via endochondral ossification. As for a method for including the endochondral ossification of the chondrocyte layer to the cortical bone tissue layer, there is a method that a porous sheet induced to the chondrocytes is cultured in a culture medium containing serum. Also at this time, the culture medium can preferably contain one or two or more kinds selected from ascorbic acid, an ascorbic acid derivative, and dexamethasone.

### [Examples]

The present invention will be described in detail below with examples, but the present invention is not limited to these examples.

### Example 1:

### 1)Taking of bone marrow from a femur and a tibia

Approximately 7×10⁷ nucleated cells were acquired from a bone marrow by cutting and removing muscles and ligaments from a femur and a tibia of a 4 week-age rabbit, cutting both ends of the femur and the tibia, washing an inside of the marrow with 10% fetal bovine serum and a αMEM culture medium, fully suspending and dispersing the bone marrow in the washing culture medium, and subjecting the washing culture medium to centrifugal separation at 300G for 5 minutes so as to separate the cells.

### 2)Culture of marrow-derived mesenchymal stem cells:

The 7×10⁷ nucleated cells taken from the marrow were seeded in a culture flask of 75cm² containing the 10% fetal bovine serum and the αMEM culture medium similar to the above, and cultured under an existence of 5% carbon dioxide at 37°C. The culture medium was changed on the 6th day so as to remove non-adherent cells. Thereafter, the culture medium was changed once every three days. Further, bFGF was added to the culture medium at the ratio of 3 ng/ml from the 5th day. As a result, the cells were proliferated almost densely on about the 10th day. The culture flask was incubated.for 5 minutes with trypsin of 0.05% and EDTA of 0.2mM so as to peel and collect the cells. The number of cells was measured by Coulter Counter (Z1 Single, produced by Beckman Coulter, Inc), and the cells were seeded on the second subculture dish at a density of 5×10³ cells/cm². The operation from the culturing of the cells under an existence of 5% carbon dioxide at 37°C to the incubating of the culture dish for 5 minutes so as to collect the cells was repeated again, and the third generation cells acquired from the second subculture dish were used.

### 3) Production of a porous bioabsorbable polymer sheet:

A porous bioabsorbable polymer sheet was produced by dissolving a polymer blend with dioxane, where the polymer blend was made by mixing poly-L-lactic acid having a molecular weight of 230,000 and a DL-lactic acid / glycolic acid copolymer having a molecular weight of 250,000 at a predetermined ratio, and freeze-drying the mixture. The obtained porous sheet had an average pore diameter of 20µm, a porosity of 52%, and a thickness of 250µm.

### 4)Production of a cartilage tissue sheet:

A cartilage tissue sheet was produced by cutting a porous bioabsorbable polymer sheet so as to have a diameter of 9 mm, seeding the mesenchymal stem cells on one side of the cut porous sheet in a dense state, applying centrifugal force of about 352G for 3 minutes in an approximately vertical direction toward the porous bioabsorbable polymer sheet from the seeded mesenchymal stem cells by a centrifugal separator (the product name: Small and Desk Type Centrifugal Separator, produced by KOKUSAN Co.Ltd.) (a radius was 15 cm, a rotation speed was 1550 rpm), and thereafter culturing the cell seeded sheet at 37°C under normal pressure for 4 weeks in a chondrogenic differentiation medium (αMEM containing ascorbate-2-phosphate of 50µg/ml, pyruvic acid of 100 µg/ml, D-(+)-glucose of 4.5 g/l, L-glutamine of 2 mM, TGF-β3 of 10 ng/ml, dexamethasone of 10⁻⁷M, and 1% ITS⁺).

### 5)Production of a bone tissue regeneration sheet:

A bone tissue regeneration sheet was produced by culturing the cartilage tissue sheet at 37°C under normal pressure for 4 weeks with an osteogenic differentiation medium (αMEM containing 10% fetal bovine serum, ascorbate-2-phosphate of 50µg/ml, L-glutamine of 2 mM, D dexamethasone of 10⁻⁷M, and β -glycerophosphate of 10mM).

### Example 2:

### 1) Mesenchymal stem cells taken from bone marrow of a human ilium:

Cells taken from bone marrow of a human ilium were suspended in 10% fetal bovine serum and a αMEM medium, and seeded on a culture dish at nucleated cells of 1×10⁷ cells/10cm diameter. The cells were cultured under an existence of 5% carbon dioxide at 37°C. The culture medium was changed on the 3rd day so as to remove non-adhesive cells (hematopoietic cells). Thereafter, the culture medium was changed once every three days. bFGF was added to the culture medium at the ratio of 3 ng/ml from the 5th day. The cells were proliferated almost densely on about the 10th day. These culture dishes were incubated for 5 minutes with trypsin (0.05%) and EDTA (0.2mM) so as to isolate the cells. The number of cells was measured by Coulter Counter (Z1 Single, produced by Beckman Coulter, Inc), and the cells were seeded on the second subculture dish at the density of 5×10³ cells/cm². The operation from the culturing of the cells under an existence of 5% carbon dioxide at 37°C to the incubating of the culture dish for 5 minutes so as to collect the cells was repeated again, and the third generation cells acquired from the second subculture dish were used.

### 2) Production of a porous bioabsorbable polymer sheet:

A porous bioabsorbable polymer sheet was produced by dissolving a DL-lactic acid / glycolic acid copolymer having a molecular weight of 250,000 with dioxane, and freeze-drying the mixture. The porous sheet had an average pore diameter of 20µm, a porosity of 52%, and a thickness of 250µm.

### 3)Production of a cartilage tissue sheet:

A cartilage tissue sheet was produced by cutting a porous bioabsorbable polymer sheet so as to have a diameter of 9 mm, seeding the mesenchymal stem cells on one side of the cut porous sheet in a dense state, applying centrifugal force of about 352G for 3 minutes in an approximately vertical direction toward the porous bioabsorbable polymer sheet from the seeded mesenchymal stem cells by a centrifugal separator (the product name: Small and Desk Type Centrifugal Separator, produced by KOKUSAN Co.Ltd.) (a radius is 15 cm and a rotation speed is 1550 rpm), and thereafter culturing the cell seeded sheet at 37°C under normal pressure for 4 weeks with a chondrogenic differentiation medium (αMEM containing ascorbate-2-phosphate of 50µg/ml, pyruvic acid of 100 µg/ml, D-(+)-glucose of 4.5 g/l, L-glutamine of 2 mM, TGF-β3 of 10 ng/ml, dexamethasone of 10⁻⁷M, and 1% ITS⁺).

### 4)Production of a bone tissue regeneration sheet:

A bone tissue regeneration sheet was produced by culturing the cartilage tissue sheet at 37°C under normal pressure for 4 weeks in an osteogenic differentiation medium (αMEM containing 10% fetal bovine serum, ascorbate-2-phosphate of 50µg/ml, L-glutamine of 2 mM, dexamethasone of 10⁻⁷M and β -glycerophosphate of 10mM). After finish of the culturing, the sheet was frozen and embedded so as to make a thin slice having a thickness of 7µm. When the thin slice was dyed with hematoxylin eosin and observed by a microscope, it was confirmed that a cortical bone like tissue layer having a thickness of about 170µm was formed following a portion invading about 40µm from one face of the sheet material, and thus the layer had a total thickness of about 210µm.

The bone tissue regeneration sheet produced by using the human mesenchymal stem cells as a raw material, which is produced by the production method of the present invention, was transplanted to a scid mouse. At a time of 8 weeks after the transplantation, a sample was taken and dyed with hematoxylin eosin, alizarin red, and human vimentin antibody. Further, m-RNA was collected from the sample and carried out an expression analysis of a type I collagen and osteocalcin, which are peculiarly seen in a bone tissue, by RT-PCR. As a result, it was observed that the sample from the scid mouse, which was transplanted under the skin of the back of the scid mouse, had a surface gloss and milk white color. Further, from the results of dying of the human vimentin positive tissue with hematoxylin eosin and alizarin red, a calcified tissue containing richly collagen was observed. Further, m-RNA expressing human type I collagen and human osteocalcin was detected and identified in the m-RNA sample extracted from the sample. Therefore, it was confirmed that the transplanted human bone tissue regeneration sheet functioned in the mouse body. Further, it was also confirmed that the obtained bone tissue regeneration sheet had the bone cells in high dense state and the bone cells were cultured on the surface and to a full depth from the surface.

### <Comparative example 1>

### 1)Taking of a bone marrow from femur and tibia:

Approximately 7×10⁷ nucleated cells were acquired from a bone marrow by cutting and removing muscles and ligaments from a femur and a tibia of a 4 week-age rat, cutted both ends of the femur and the tibia, washing an inside of the marrow with 10% fetal bovine serum and a αMEM culture medium, fully suspending and dispersing the bone marrow in the washing medium, and subjecting the washing medium to centrifugal separation at 300G for 5 minutes so as to separate cells.

### 2)Culture of marrow-derived mesenchymal stem cells:

The 7×10⁷ nucleated cells taken from the rat marrow were seeded in a culture flask of 75cm² containing the 10% fetal bovine serum and the αMEM culture medium similar to the above, and cultured under an existence of 5% carbon dioxide at 37°C. The culture medium was changed on the 5th day so as to remove non-adheherent cells. Thereafter, the culture medium was changed once every three days. Further, bFGF was added to the culture medium at the ratio of 3 ng/ml from the 5th day. As a result, the cells were proliferated almost densely on about the 10th day. The culture flask was incubated for 5 minutes with trypsin of 0.05% and EDTA of 0.2mM so as to peel and collect the cells. The number of cells was measured by Coulter Counter (Z1 Single, produced by Beckman Coulter, Inc), and the cells were seeded on the second subculture dish at a density of 5×10³ cells/cm². The operation from the culturing of the cells under an existence of 5% carbon dioxide at 37°C to the incubating of the culture dish for 5 minutes so as to collect the cells was repeated again, and the third generation cells acquired from the second subculture dish were used.

### 3) Production of a porous bioabsorbable polymer sheet:

A porous sheet was produced by dissolving a polymer blend with dioxane, where the polymer blend was obtained by mixing poly-L-lactic acid having a molecular weight of 230,000 and a DL-lactic acid / glycolic acid copolymer having a molecular weight of 250,000 at a predetermined ratio, and freeze-drying the mixture. The porous sheet had an average pore diameter of 20µm, a porosity of 52%, and a thickness of 250µm.

### 4)Production of an osteoblast tissue regeneration sheet:

An osteoblast tissue regeneration sheet was produced by cutting a porous bioabsorbable polymer sheet so as to have a diameter of 9 mm, seeding the mesenchymal stem cells on the porous sheet so as to have a concentration of 2x10⁴ cells/cm², and culturing the porous body at 37°C under normal pressure for 4 weeks with an osteogenic differentiation medium (αMEM containing 10% fetal bovine serum, ascorbate-2-phosphate of 50µg/ml, L-glutamine of 2 mM, dexamethasone of 10⁻⁷M, and β- glycerophosphate of 10mM). After finish of the culturing, the sheet was frozen and embedded so as to produce a thin slice having a thickness of 7µm. When the thin slice was dyed with hematoxylin eosin and observed by a microscope, it was confirmed that a bone tissue layer having a thickness of about 10µm was formed following a portion invading about 40µm from one face of the sheet material, and thus the bone tissue layer only had a very thin thickness of about 50µm in total.

## Claims

1. A production method of a bone tissue regeneration sheet, the method comprising steps of:
forming a chondrocyte layer having a thickness of 200 µm or more on one side of a porous bioabsorbable polymer sheet; and
making endochondral ossification of the chondrocytes so as to form a cortical bone tissue layer having a thickness of 200 µm or more.

2. The production method of a bone tissue regeneration sheet as claimed in claim 1,
wherein the method for forming a chondrocyte layer having a thickness of 200 µm or more on one side of a porous bioabsorbable polymer sheet comprises steps of :
seeding chondrocytes or stem cells for differentiating to chondrocytes on one side of a porous bioabsorbable polymer sheet;
taking the seeded porous body into a culture medium;
applying centrifugal force of 100 to 1000G to the culture medium for a predetermined time so as to aggregate the chondroblasts or the stem cells for differentiating to chondrocytes;
culturing thereafter the aggregated cells in a culture medium not containing serum but containing one or two or more kinds selected from ascorbic acid, an ascorbic acid derivative, and dexamethasone without applying centrifugal force to the culture medium so as to form a chondrocyte layer having a thickness of 200 µm or more.

3. The production method of a bone tissue regeneration sheet as claimed in claim 1 or 2,
wherein the method for making endochondral ossification of the chondrocytes so as to form a cortical bone tissue layer having a thickness of 200 µm comprises a step of culturing the chondrocyte layer of 200 µm or more in a culture medium containing serum so as to make endochondral ossification of the chondrocyte layer to form a cortical bone tissue layer of 200 µm or more.

4. A bone tissue regeneration sheet having a cortical bone tissue having a thickness of 200µm or more on one side of a porous bioabsorbable polymer sheet, obtainable by the production method of a bone tissue regeneration sheet according to any one of claims 1 to 3.

## Patentansprüche

1. Herstellungsverfahren für eine Knochengeweberegenerierungsfolie, wobei das Verfahren die Schritte umfaßt:
Bilden einer Chrondrozytenschicht, die eine Dicke von 200 µm oder mehr aufweist, auf einer Seite einer porösen, bioabsorbierbaren Polymerfolie, und
Verursachen von endochondraler Ossifikation der Chondrozyten, um eine kortikale Knochengewebsschicht zu bilden, die eine Dicke von 200 µm oder mehr aufweist.

2. Herstellungsverfahren für eine Knochengeweberegenerierungsfolie nach Anspruch 1, wobei das Verfahren zum Bilden einer Chondrozytenschicht, die eine Dicke von 200 µm oder mehr aufweist, auf einer Seite einer porösen, bioabsorbierbaren Polymerfolie die Schritte umfaßt:
Aussähen von Chondrozyten oder Stammzellen für die Differenzierung zu Chondrozyten auf eine Seite einer porösen, bioabsorbierbaren Polymerfolie,
Aufnehmen des besäten porösen Körpers in ein Kulturmedium,
Anwenden einer Zentrifugalkraft von 100 bis 1000G auf das Kulturmedium für eine vorbestimmte Zeit, so daß die Chondroblasten oder die Stammzellen für die Differenzierung zu Chondrozyten aggregiert werden,
danach Kultivieren der aggregierten Zellen in einem Kulturmedium, das kein Serum enthält, sondern ein oder zwei oder mehr Arten, ausgewählt aus Ascorbinsäure, einem Ascorbinsäurederivat und Dexamethason, enthält, ohne eine Zentrifugalkraft auf das Kulturmedium anzuwenden, um eine Chondrozytenschicht, die eine Dicke von 200 µm oder mehr aufweist, zu bilden.

3. Herstellungsverfahren für eine Knochengeweberegenerierungsfolie nach Anspruch 1 oder 2, wobei das Verfahren zum Verursachen von endochondraler Ossifikation der Chondrozyten, um eine kortikale Knochengewebsschicht zu bilden, die eine Dicke von 200 µm aufweist, einen Schritt des Kultivierens der Chondrozytenschicht von 200 µm oder mehr in einem Kulturmedium, das Serum enthält, umfaßt, um endochondrale Ossifikation der Chondrozytenschicht zu verursachen, um eine kortikale Knochengewebsschicht von 200 µm oder mehr zu bilden.

4. Knochengeweberegenerierungsfolie mit einem kortikalen Knochengewebe mit einer Dicke von 200 µm oder mehr auf einer Seite einer porösen bioabsorbierbaren Polymerfolie, erhältlich durch das Herstellungsverfahren für eine Knochengeweberegenerierungsfolie nach einem der Ansprüche 1 bis 3.

## Revendications

1. Méthode de production d'une feuille de régénération de tissu osseux, la méthode comprenant les étapes de :
former une couche de chondrocytes ayant une épaisseur de 200 µm ou plus sur un côté d'une feuille de polymère bioabsorbable poreuse ; et
réaliser l'ossification endochondrale des chondrocytes de manière à former une couche de tissu osseux cortical ayant une épaisseur de 200 µm ou plus.

2. La méthode de production d'une feuille de régénération de tissu osseux telle que revendiquée à la revendication 1,
dans laquelle la méthode pour former une couche de chondrocytes ayant une épaisseur de 200 µm ou plus sur un côté d'une feuille de polymère bioabsorbable poreuse comprend les étapes de :
ensemencer des chondrocytes ou des cellules souches pour leur différenciation en chondrocytes sur un côté d'une feuille de polymère bioabsorbable poreuse ;
introduire le corps poreux ensemencé dans un milieu de culture ;
appliquer une force centrifuge de 100 à 1000 G au milieu de culture pendant une durée prédéterminée de manière à agréger les chondroblastes ou les cellules souches en vue de leur différenciation en chondrocytes ;
mettre en culture par la suite les cellules agrégées dans un milieu de culture ne contenant pas de sérum, mais contenant un ou deux ou plusieurs types sélectionnés parmi de l'acide ascorbique, un dérivé d'acide ascorbique et de la dexaméthasone sans appliquer de force centrifuge au milieu de culture de manière à former une couche de chondrocytes ayant une épaisseur de 200 µm ou plus.

3. La méthode de production d'une feuille de régénération de tissu osseux telle que revendiquée à la revendication 1 ou 2,
dans laquelle la méthode pour réaliser une ossification endochondrale des chondrocytes de manière à former une couche de tissu osseux cortical ayant une épaisseur de 200 µm comprend une étape de mise en culture de la couche de chondrocytes de 200 µm ou plus dans un milieu de culture contenant du sérum, de manière à réaliser une ossification endochondrale de la couche de chondrocytes pour former une couche de tissu osseux cortical de 200 µm ou plus.

4. Une feuille de régénération de tissu osseux ayant un tissu osseux cortical ayant une épaisseur de 200 µm ou plus sur un côté d'une feuille de polymère bioabsorbable poreuse, qui peut être obtenue par la méthode de production d'une feuille de régénération de tissu osseux selon l'une quelconque des revendications 1 à 3.
